# EUROPEAN PATENT APPLICATION

(11) **EP 1 354 949 A1**
(43) Date of publication of application: **22.10.2003**
(21) Application number: 01271888.8
(22) Date of filing: 26.12.2001
(51) Int. Cl.: C12N 15/12, C12N 5/10, C07K 14/50, C12P 21/02, C12Q 1/68, A61K 35/76, A61K 38/00, A61K 48/00, A61P 3/12

(54) **HUMAN FGF23 PROTEIN MUTANT LOWERING BLOOD PHOSPHORUS LEVEL**

(30) Priority: 26.12.2000 JP 2000396316; 29.05.2001 JP 2001161370
(71) Applicant: CHUGAI SEIYAKU KABUSHIKI KAISHA, Tokyo, 115-8543 (JP)
(72) Inventor: ITOH, H., c/o CHUGAI SEIYAKU KABUSHIKI KAISHA, Gotenba-shi, Shizuoka 412-8513 (JP); FUKUSHIMA, N., c/o CHUGAI SEIYAKU KABUSHIKI KAISHA, Gotenba-shi, Shizuoka 412-8513 (JP); SAITO, H., c/o CHUGAI SEIYAKU KABUSHIKI KAISHA, Gotenba-shi, Shizuoka 412-8513 (JP); KUSANO, K., c/o CHUGAI SEIYAKU KABUSHIKI KAISHA, Gotenba-shi, Shizuoka 412-8513 (JP)
(74) Representative: Bassil, Nicholas Charles
(86) International application number: JP0111482
(87) International publication number: WO02052009

(57) **Abstract**

A full-length cDNA encoding the human FGF23 protein was isolated, and mutants having a single amino acid substitution in the cDNA were constructed using the mutagenesis method. These mutants had the effect of decreasing the phosphorus level in the blood when expressed *in vivo.* These mutants and the DNAs encoding them are expected to be useful as therapeutic agents, or employed for gene therapy against hyperphosphatemia.

## Description

### Technical Field

The present invention relates to FGF23 protein mutants that decrease the phosphorus levels in the blood, and use of these mutants.

### Backqround Art

FGF23 is a gene cloned by Ito et al. at Kyoto University, and its expression in brain has been confirmed (Yamashita T. et al. (2000) Biochem. Biophys. Res. Commun. 277: 494-498; W001/66596). Furthermore, Luethy et al. have cloned the FGF23 gene to produce a transgenic mouse that expresses the gene, and analyzed the phenotype of the mouse (W001/61007).

On the other hand, based on a genetic pedigree analysis of patients suffering from rickets (a congenital hypophosphatemia), the disease was reported to be caused by mutations (R176Q, R179Q, R179W) of FGF23 (The ADHR Consortium (2000) Nat. Genet. 26: 345-348).

However, this report does not mention that the protein or the full-length cDNA was obtained, nor does it reveal the causal relationship between the above-mentioned mutation observed in the FGF23 gene and hypophosphatemia.

### Disclosure of the Invention

The present invention was accomplished in view of the above observations. Specifically, the objectives of the present invention include providing FGF23 protein mutants that decrease the phosphorus level in the blood, DNAs encoding these mutants, and the use of these mutants and DNAs.

The present inventors carried out extensive studies to obtain the above-mentioned objectives, and aimed to produce mutants of the FGF23 protein expected to decrease the phosphorous level in the blood. First, the full-length cDNA of the human FGF23 gene was isolated, and then a DNA encoding the mutant was synthesized using the PCR method and cloned into an expression vector. The expression vector was introduced into a mouse via intravenous administration to express the mutant *in vivo,* and then the blood phosphorus level of the mouse was measured. The results showed that the phosphorus level in the mouse blood significantly decreased due to the expression of the FGF23 mutant.

Specifically, in addition to the successful production of the FGF23 protein mutants, the present inventors succeeded in decreasing the phosphorus level in mouse blood using these mutants, and finally accomplished the objectives of the present invention. Since the FGF23 protein mutants of this invention have the ability to decrease the phosphorus level in the blood as described above, they are highly expected to serve as pharmaceutical agents for treating hyperphosphatemia.

The present invention relates to FGF23 protein mutants that decrease the phosphorus level in the blood, DNAs encoding these mutants, and use thereof. More specifically, the present invention provides the following:
(1) a DNA encoding a protein comprising the amino acid sequence of SEQ ID NO: 2 having a mutation selected from the group of mutations of arginine at position 176 to glutamine, arginine at position 179 to glutamine, and arginine at position 179 to tryptophan;
(2) a DNA encoding a fragment having at least the amino acid sequence from position 1 to position 190 of a protein comprising the amino acid sequence of SEQ ID NO: 2 that has a mutation selected from the group of mutations of arginine at position 176 to glutamine, arginine at position 179 to glutamine, and arginine at position 179 to tryptophan;
(3) a vector into which the DNA according to (1) or (2) is inserted;
(4) a transformed cell harboring the DNA according to (1) or (2), or the vector according to (3) ;
(5) a protein comprising the amino acid sequence of SEQ ID NO: 2 having a mutation selected from the group of mutations of arginine at position 176 to glutamine, arginine at position 179 to glutamine, and arginine at position 179 to tryptophan;
(6) a fragment having at least the amino acid sequence from position 1 to position 190 of a protein comprising the amino acid sequence of SEQ ID NO: 2 that has a mutation selected from the group of mutations of arginine at position 176 to glutamine, arginine at position 179 to glutamine, and arginine at position 179 to tryptophan;
(7) a method for producing the protein according to (5) or (6), wherein the method comprises the steps of culturing the transformed cell according to (4), and collecting expressed protein from the transfected cell or the culture supernatant thereof;
(8) a pharmaceutical composition for decreasing the blood phosphorus level, which comprises the DNA according to (1) or (2), the vector according to (3), or the protein according to (5) or (6);
(9) the pharmaceutical composition according to (8), which does not influence the blood calcium level;
(10) the pharmaceutical composition according to (8) or (9) for treating hyperphosphatemia; and
(11) a method for treating hyperphosphatemia, which comprises the step of administering the DNA according to (1) or (2) to a patient.

The present invention provides DNAs encoding the FGF23 protein mutants that decrease the blood phosphorus level. The mutants encoded by the DNAs of this invention include mutants wherein the arginine residue at position 176 is substituted with glutamine, the arginine residue at position 179 is substituted with glutamine or the arginine at position 179 is substituted with tryptophan in the amino acid sequence of the human FGF23 protein of SEQ ID NO: 2 (hereinafter, these mutants are referred to as "R176Q mutant", "R179Q mutant", and "R179W mutant", respectively, and all of them together are referred to as "FGF mutants"). Among these mutants, the R176Q mutant and R179Q mutant are preferable, and the R179Q mutant is most preferable.

The present invention also provides fragments of these FGF mutants. Preferable fragments comprise at least the amino acid sequence from position 1 to position 190 of the FGF mutant.

The FGF23 mutants of this invention have the function to decrease the phosphorus level in the blood. Therefore, the FGF23 mutants are expected to exert therapeutic and preventive effects against diseases caused by the presence of high levels of phosphorus in the blood. According to a preferred embodiment of the present invention, the FGF23 mutant does not influence the blood calcium level.

An example of a disease for which the therapeutic and preventive effects may be expected is hyperphosphatemia. Hyperphosphatemia is generally developed because of decrease in PO₄ excretion from the kidney. Advanced renal failure (glomerular filtration rate (GFR) of less than 20 mL/min) causes decrease of excretion that is sufficient to lead to the increase of plasma PO₄. Even without the cause of renal failure, pseudohypoparathyroidism or hypoparathyroidism may also induce disorders in renal PO₄ excretion. Hyperphosphatemia can also occur due to excess administration of oral PO₄, or sometimes due to the overuse of enema containing phosphate salts. Furthermore, hyperphosphatemia may occur as a result of migration of intracellular PO₄ to the cell exterior. Such migration frequently occurs in diabetic ketoacidosis (regardless of systemic PO₄ loss), bruise, non-traumatic rhabdomyolysis, systemic infection and tumor lysis syndrome. Moreover, hyperphosphatemia plays a critical role in the onset of secondary hyperparathyroidism, and the onset of renal osteodystrophy in patients under dialysis treatment for a long period.

The DNAs of the present invention are utilized for *in vivo* and *in vitro* production of mutants of the present invention as described below. In addition, they may be applicable in gene therapy against diseases due to high blood phosphorus level. The DNAs of this invention can take any form as long as they encode the mutants of this invention. For example, it does not matter whether the DNA is a cDNA synthesized from mRNA, is genomic DNA, or is chemically synthesized. Furthermore, DNAs having arbitrary nucleotide sequences based on the degeneracy of the genetic code are included in the DNAs of the present invention as long as they encode the mutants of the invention.

The DNAs of the present invention can be produced by modifying a human FGF23 cDNA. The human FGF23 cDNA can be prepared by methods well known to those skilled in the art. For example, it can be prepared by producing a cDNA library from cells expressing FGF23 and then performing hybridization using a portion of the FGF23 cDNA sequence (SEQ ID NO: 1) as a probe. For example, the cDNA library can be prepared, by a method described in the literature (Sambrook, J. et al., Molecular Cloning, Cold Spring Harbor Laboratory Press (1989)), or a commercially available DNA library may be used. Furthermore, the library can be prepared as follows: (1) preparing RNA from cells expressing FGF23; (2) synthesizing cDNA using reverse transcriptase; (3) synthesizing an oligo-DNA based on the cDNA sequence of FGF23 (SEQ ID NO: 1); and (4) conducting PCR using this oligo-DNA as a primer to amplify the cDNA encoding the polypeptide of this invention.

More specifically, mRNA may first be isolated from a cell, tissue or organ that expresses FGF23. Known methods can be used to isolate mRNA. For instance, total RNA can be prepared by guanidine ultracentrifugation (Chirgwinj. M. et al., Biochemistry 18:5294-5299 (1979)) or by the AGPC method (Chomczynski P. and Sacchi N., Anal. Biochem. 162:156-159 (1987)), and mRNA is purified from the total RNA using an mRNA Purification Kit (Pharmacia) , etc. Alternatively, mRNA may be directly prepared using a QuickPrep mRNA Purification Kit (Pharmacia).

The mRNA obtained is used to synthesize cDNA using reverse transcriptase. cDNA may be synthesized using a kit such as the AMV Reverse Transcriptase First-strand cDNA Synthesis Kit (Seikagaku Kogyo). Alternatively, cDNA may be synthesized and amplified following the 5'-RACE method (Frohman M.A. et al., Proc. Natl. Acad. Sci . U.S.A. 85:8998-9002 (1988); Belyavsky A. et al., Nucleic Acids Res. 17:2919-2932 (1989)) that uses primers prepared based on the sequence described in SEQ ID NO: 1, 5'-Ampli FINDER RACE Kit (Clontech), and polymerase chain reaction (PCR).

A desired DNA fragment is prepared from the PCR products and linked to a vector DNA. The recombinant vector is used to transform *Escherichia coli,* etc., and the desired recombinant vector is prepared from a selected colony. The nucleotide sequence of the desired DNA can be verified by conventional methods, such as dideoxynucleotide chain termination.

Specifically, human FGF23 cDNA can be obtained, for example, by the method described in Example 1 below.

Modification of a human FGF23 cDNA for producing a FGF23 mutant of the present invention can be carried out by the DNA mutagenesis technique commonly performed by those skilled in the art. For example, the modification can be carried out by the method indicated in Example 2 below.

The present invention also provides mutants encoded by the above-mentioned DNAs of the present invention. The mutants of this invention may show differences in their amino acid sequences, molecular weights, isoelectric points, or the presence or form of sugar chains and will depend on the cell or host producing the mutants, or the method of production described below. However, as long as the obtained mutants have the ability to decrease the phosphorus level in the blood, they are included in the present invention. For example, when a mutant of the present invention is expressed in a procaryotic cell, such as *E*. *coli,* a methionine residue will be added to the N-terminus of the amino acid sequence of the original mutant. Such a mutant is also included in this invention.

A mutant of the present invention can be prepared as a recombinant polypeptide by methods well known to those skilled in the art. The recombinant polypeptide can be prepared by inserting a DNA encoding the mutant of this invention into an appropriate expression vector, collecting a transformant obtained by transfecting the vector into an appropriate host cell, and purifying the polypeptide after obtaining the extract by chromatography, such as ion exchange chromatography, reverse phase chromatography, gel filtration chromatography, or affinity chromatography wherein antibodies against the mutant of this invention are fixed onto a column, or by combining a plurality of these columns.

Furthermore, when a mutant of the present invention is expressed in a host cell (for example, animal cell, *E. coli,* etc.) as a polypeptide fused with glutathione S-transferase protein or a recombinant polypeptide added with a plurality of histidines, the expressed recombinant polypeptide can be purified using a glutathione column or a nickel column. After purifying the fused polypeptide, regions other than the desired mutant can be removed from the fused polypeptide as necessary by cleaving it with thrombin, factor Xa, etc.

The present invention also provides vectors into which a DNA of the present invention is inserted. The vectors of the present invention are useful in maintaining the DNAs of the present invention within the host cell, or expressing a mutant of the present invention.

When *E*. *coli* is used as the host cell, there is no limitation other than that the vector should have an "ori", and a marker gene. The "ori" for amplifying and mass-producing the vector in *E*. *coli* (e.g., JM109, DH5α, HB101, or XL1Blue). And the marker gene for selecting the transformed *E*. *coli* (e.g., a drug-resistance gene selected by a drug (e.g., ampicillin, tetracycline, kanamycin, or chloramphenicol)). For example, M13-series vectors, pUC-series vectors, pBR322, pBluescript, pCR-Script, and such can be used. Besides the vectors, pGEM-T, pDIRECT, pT7, etc. can also be used for the subcloning and excision of the cDNA. When a vector is used to produce a mutant of the present invention, an expression vector is especially useful. When the expression vector is expressed, in *E*. *coli,* it should have the above characteristics in order to be amplified in *E*. *coli.* Additionally, when *E*. *coli,* such as JM109, DH5α, HB101 or XL1-Blue are used as the host cell, the vector should have a promoter, e.g., lacZ promoter (Ward et al. (1989) Nature 341:544-546; (1992) FASEB J. 6:2422-2427), araB promoter (Better et al. (1988) Science 240:1041-1043), or T7 promoter, that can efficiently promote the expression of the desired gene in *E*. *coli.* Other examples of the vectors are pGEX-5X-1 (Pharmacia), "QIAexpress system" (QIAGEN), pEGFP, and pET (for this vector, BL21, a strain expressing T7 RNA polymerase, is preferably used as the host).

Furthermore, the vector may comprise a signal sequence to secrete the polypeptide. For producing the polypeptide into the periplasm of *E*. *coli,* the pelB signal sequence (Lei, S. P. et al. (1987) J. Bacteriol. 169:4379) may be used as the signal sequence for secretion of the polypeptide. For example, the calcium chloride method or electroporation may be used to introduce the vector into host cells.

As vectors used to produce the mutants of the present invention, expression vectors derived from mammals (e.g., pCDNA3 (Invitrogen), pEGF-BOS (Nucleic Acids Res. (1990) 18(17) :5322), pEF, pCDM8) , insect cells (e.g., "Bac-to-BAC baculovirus expression system" (GIBCO-BRL) , pBacPAK8), plants (e.g., pMH1, pMH2), animal viruses (e.g., pHSV, pMV, pAdexLcw) , retroviruses (e.g., pZIPneo), yeasts (e.g., "Pichia Expression Kit" (Invitrogen), pNV11 SP-Q01), and *Bacillus subtilis* (e.g. , pPL608, pKTH50) can be mentioned other than those derived from *E.coli.*

In order to express proteins in animal cells, such as CHO, COS, and NIH3T3 cells, the vector must have a promoter necessary for expression in such cells (e.g., SV40 promoter (Mulligan et al. (1979) Nature 277:108), MMLV-LTR promoter, EF1α promoter (Mizushima et al. (1990) Nucleic Acids Res. 18:5322) , CMV promoter, etc.). It is more preferred if the vector additionally has a marker gene for selecting transformants (for example, a drug resistance gene selected by a drug (e.g., neomycin, G418, etc.)). Examples of vectors with such characteristics include pMAM, pDR2, pBK-RSV, pBK-CMV, pOPRSV, pOP13, etc.

Furthermore, in order to stably express the gene and to amplify the copy number in cells, the method using CHO cells deficient in nucleic acid synthetic pathways as the host, incorporating into the CHO cells a vector (such as pCHOI) having a DHFR gene that compensates for the deficiency, and amplifying the vector with methotrexate (MTX) can be used. Furthermore, for transiently expressing a gene, the method that transforms COS cells that have the gene for SV40 T antigen on the chromosome with a vector (such as pcD) having the SV40 replication origin can be mentioned. The replication origin may be that of a polyomavirus, adenovirus, bovine papilloma virus (BPV), etc. In addition, to amplify the gene copy number in the host cells, selection markers such as the aminoglycoside transferase (APH) gene, thymidine kinase (TK) gene, *E*. *coli* xanthine-guanine phosphoribosyl transferase (Ecogpt) gene, and the dihydrofolate reductase (dhfr) gene may be incorporated into the expression vector.

Examples of expressing a DNA of the present invention in animals include inserting a DNA of the invention into an appropriate vector and introducing the vector into a living body by the retrovirus method, liposome method, cationic liposome method, adenovirus method, etc. Thus, it is possible to perform gene therapy of diseases caused by the presence of high levels of phosphorus in the blood. The vectors used in these methods include, but are not limited to, adenovirus vectors (e.g., pAdexlcw), retrovirus vectors (e.g., pzIPneo) , etc. General techniques for gene manipulation, such as insertion of the DNA of the invention into a vector, can be performed according to conventional methods (Molecular Cloning, 5.61-5.63). Administration to the living body may be performed according to the ex *vivo* method or the *in vivo* method.

The present invention also provides host cells into which a vector of the present invention has been introduced. Host cells into which the vectors of the invention are introduced are not particularly limited. For example, *E*. *coli,* and various animal cells can be used. The host cell of the present invention can be used, for example, as a production system to produce and express a protein of the present invention. Protein production systems include *in vitro* and *in vivo* systems. Such production systems using eukaryotic cells or prokaryotic cells can be given as *in vitro* production systems.

For example, as eukaryotic host cells, animal cells, plant cells and fungi cells can be used. Mammalian cells, for example, CHO (J.Exp.Med. (1995) 108:945), COS, 3T3, myeloma, BHK (baby hamster kidney), HeLa, Vero, amphibian cells (e.g., platanna oocytes (Valle et al. (1981) Nature 291:358-340) , and insect cells (e.g., Sf9, Sf21, Tn5) are known as animal cells. Among CHO cells, those deficient in the DHFR gene, dhfr-CHO (Proc. Natl. Acad. Sci. USA (1980) 77:4216-4220) and CHO K-1 (Proc. Natl. Acad. Sci. USA (1968) 60:1275) are particularly preferable. Among animal cells, CHO cells are particularly preferable for mass expression. A vector can be introduced into a host cell by, for example, the calcium phosphate method, the DEAE-dextran method, methods using cationic liposome DOTAP (Boehringer-Mannheim), electroporation, lipofection, etc.

Plant cells originating from *Nicotiana tabacum* are known as polypeptide producing systems and may be used as callus cultures. As fungal cells, yeast cells such as *Saccharomyces,* including *Saccharomyces cerevisiae,* or filamentous fungi such as *Aspergillus,* including *Aspergillus niger,* are known.

Useful prokaryotic cells for peptide production include bacterial cells. Bacterial cells such as *E*. *coli* (for example, JM109, DH5α, HB101, etc.) as well as *Bacillus subtilis* are known to be useful for peptide production.

These cells are transformed by a desired DNA, and the resulting transformants are cultured *in vitro* to obtain the polypeptide. Transformants can be cultured using known methods. For example, culture medium such as DMEM, MEM, RPMI1640, or IMDM may be used with or without serum supplements such as fetal calf serum (FCS) as culture medium for animal cells. The pH of the culture medium is preferably between about 6 and about 8. Such cells are typically cultured at about 30°C to about 40°C for about 15 hr to about 200 hr, and the culture medium may be replaced, aerated or stirred if necessary.

Animal and plant hosts may be used for *in* vivo polypeptide production. For example, a DNA encoding a mutant of the present invention can be introduced into an animal or plant host. The mutant is produced *in vivo* and then recovered. These animal and plant hosts are included in the "host" of the present invention.

Animals to be used for the production system described above include mammals and insects. Mammals such as goats, pigs, sheep, mice, and cattle may be used (Vicki Glaser (1993) SPECTRUM Biotechnology Applications). Alternatively, the mammals may be transgenic animals.

For instance, a DNA encoding a mutant of the present invention may be prepared as a fusion gene with a gene such as goat β casein gene that encodes a polypeptide specifically produced into milk. DNA fragments comprising the fusion gene are injected into goat embryos, which are then introduced back to female goats. The mutant of this invention can be obtained from milk produced by the transgenic goats (i.e., those born from the goats that had received the modified embryos) or from their offspring. To increase the amount of milk containing the polypeptides produced by transgenic goats, appropriate hormones may be administered (Ebert, K. M. et al., (1994) Bio/Technology 12:699-702).

Alternatively, insects such as the silkworm may be used. Baculoviruses into which a DNA encoding a mutant of this invention has been inserted can be used to infect silkworms, and the mutant can be recovered from the body fluid (Susumu M. et al., (1985) Nature 315:592-594) .

Amongst plants, tobacco can be used. When using tobacco, a DNA encoding a mutant of this invention may be inserted into a plant expression vector, such as pMON 530, which is introduced into bacteria, such *as Agrobacterium tumefaciens.* Then, the bacteria are used to infect tobacco such as *Nicotiana tabacum,* and the desired mutant is recovered from the leaves (Julian K. -C. Ma et al., (1994) Eur. J. Immunol. 24:131-138).

A mutant of the present invention obtained as above may be isolated from inside or outside of host cells (e.g., medium), and purified as a substantially pure homogeneous polypeptide. The method for polypeptide isolation and purification is not limited to any specific method. In fact, any standard method may be used. For instance, column chromatography, filters, ultrafiltration, salting out, solvent precipitation, solvent extraction, distillation, immunoprecipitation, SDS-polyacrylamide gel electrophoresis, isoelectric point electrophoresis, dialysis, and recrystallization may be appropriately selected and combined to isolate and purify the polypeptide.

For chromatography, for example, affinity chromatography, ion-exchange chromatography, hydrophobic chromatography, gel filtration chromatography, reverse phase chromatography, adsorption chromatography, etc. may be used (Strategies for Protein Purification and Characterization: A Laboratory Course Manual. Ed. Daniel R. Marshak et al., Cold Spring Harbor Laboratory Press (1996)). These chromatographies may be performed by liquid chromatographies such as HPLC and FPLC. Thus, the present invention provides highly purified mutants produced by the above methods.

A mutant of the present invention may be optionally modified or partially deleted by treating it with an appropriate protein-modifying enzyme before or after purification. For example, trypsin, chymotrypsin, lysylendopeptidase, protein kinase, glucosidase, etc. are used as protein-modifying enzymes.

The present invention further provides pharmaceutical compounds to decrease the phosphorus level in the blood, which compounds comprise a mutant of the invention, a DNA encoding the mutant, or a vector into which the DNA is introduced.

When using a mutant of this invention as a pharmaceutical agent for humans and other animals, such as mice, rats, guinea-pigs, rabbits, chicken, cats, dogs, sheep, pigs, cattle, monkeys, baboons, and chimpanzees, the mutant can be directly administered to a subject or administered as a pharmaceutical compound that is formulated using known pharmaceutical preparation methods. For example, according to the utility contemplated, the drugs can be taken orally as sugarcoated tablets, capsules, elixirs and microcapsules, or non-orally in the form of injections of sterile solutions or suspensions with water or any other pharmaceutically acceptable liquid. For example, the compounds can be mixed with pharmacologically acceptable carriers or medium, specifically, sterilized water, physiological saline, plant-oil, emulsifiers, solvents, surfactants, stabilizers, flavoring agents, excipients, vehicles, preservatives and binders, in a unit dose form required for generally accepted drug implementation. The amount of active ingredients in these preparations makes a suitable dosage within the indicated range acquirable.

Examples of additives that can be mixed to tablets and capsules are, binders such as gelatin, corn starch, tragacanth gum and arabic gum; excipients such as crystalline cellulose; swelling agents such as corn starch, gelatin and alginic acid; lubricants such as magnesium stearate; sweeteners such as sucrose, lactose or saccharin; flavoring agents such as peppermint, Gaultheria adenothrix oil and cherry. When the unit dosage form is a capsule, a liquid carrier, such as oil, can also be included in the above ingredients. Sterile composites for injections can be formulated following normal drug implementations using vehicles such as distilled water used for injections.

Physiological saline, glucose, and other isotonic liquids including adjuvants, such as D-sorbitol, D-mannnose, D-mannitol, and sodium chloride, can be used as aqueous solutions for injections. These can be used in conjunction with suitable solubilizers, such as alcohol, specifically ethanol, polyalcohols such as propylene glycol and polyethylene glycol, non-ionic surfactants, such as Polysorbate 80^{TM} and HCO-50.

Sesame oil or Soy-bean oil can be used as a oleaginous liquid and may be used in conjunction with benzyl benzoate or benzyl alcohol as a solubilizer; may be formulated with a buffer such as phosphate buffer and sodium acetate buffer; a pain-killer such as procaine hydrochloride; a stabilizer such as benzyl alcohol, phenol; and an anti-oxidant. The prepared injection is filled into a suitable ampule.

Methods well known to one skilled in the art may be used to administer a pharmaceutical compound to patients. Examples include, intraarterial, intravenous, subcutaneous injections, intranasal, transbronchial, intramuscular,percutaneous and oral administration. The dosage varies according to the body-weight and age of the patient and the administration method; however, one skilled in the art can suitably select the dosage.

The dose of the mutants of the invention may vary depending on the subject, target organ, symptoms, and administration methods, but may be, in general, about 100 µg to about 20 mg per day for a normal adult (body weight: 60 kg).

Although varying according to the subject, target organ, symptoms and method of administration, a single dose of a compound for parenteral administration is preferable, when administered intravenously to normal adults (60 kg body weight) in the form of injection and in the range of about 0.01 mg to about 30 mg, preferably about 0.1 mg to about 20 mg, and more preferably about 0.1 mg to about 10 mg per day. Doses converted to 60 kg body weight or per body surface area can be administered to other animals.

Furthermore, when using a DNA of the present invention as a pharmaceutical composition, it may be inserted into a vector that ensures expression of the DNA of this invention *in vivo* as mentioned above, and introduced into a living body, for example, by the retrovirus method, liposome method, cationic liposome method, adenovirus method, etc. In this manner, gene therapy can be performed against diseases caused by high blood phosphorus level. The *ex vivo* method and *in vivo* method can be used for the administration into the living body.

### Brief Description of the Drawings

Fig. 1 is a graph showing the effects of the FGF23 mutants on the inorganic phosphorus level in the serum. Inorganic phosphorus in the serum was measured for 4 days after introducing the DNA vectors. The data are shown as means ± SEM. The numbers in the columns indicate the number of animals. * P<0.05 vs. MOCK control (unpaired *t*-test).
Fig. 2 is a graph showing the effect of FGF23 on the inorganic phosphorus level in the serum. Inorganic phosphorus in the serum was measured for 4 days after the introduction of the DNA vectors. The data are shown as means ± SEM. The numbers in the columns indicate the number of animals. * P<0.05 vs. MOCK control (unpaired *t*-test).
Fig. 3 is a graph showing the effects of FGF23 and FGF23 mutant on the 1α,25(OH)₂D₃ level in the serum. 1α,25(OH)₂D₃ in the serum was measured for 4 days after introducing the DNA vectors. Serum obtained from 3 animals were mixed and used for the measurements (n=2, 6 animals/group). The data are shown as means.
Fig. 4 is a graph showing the effect of FGF23 mutant on the phosphorus transport activity of brush border membrane vesicles isolated from the kidney. The Pi uptake (for 4 days after introduction) of renal bush border membrane vesicles of a naked DNA vector introduced mouse was measured. Kidneys obtained from 2 animals were used for the measurements (n=3, 6 animals/group). The data are shown as means ± SEM.
Fig. 5 is a photograph showing the result of SDS-PAGE analysis (Coomassie brilliant blue (CBB) stained) of FGF-23 (mutant) M2-F. M denotes the molecular weight marker (BIO-RAD Laboratories, broad range).
Fig. 6 is a graph showing the serum phosphorus level decreasing effect of C-terminus deleted M2FGF23 mutants. "MOCK" indicates a mouse introduced with the parent vector, pCAGGS. "Normal" indicates a normal mouse.
Fig. 7 is a graph showing the effect of PTH (1-34) and M2FGF23 to decrease serum phosphorus in the TPTX rat.
Fig. 8 is a graph showing the effect of PTH (1-34) and M2FGF23 on serum calcium in the TPTX rat.
Fig. 9 is a graph showing the effect of PTH (1-34) and M2FGF23 on renal phosphorus excretion in the TPTX rat.

### Best Mode for Carrying out the Invention

Hereinafter, the present invention is specifically illustrated with reference to Examples, but is not to be construed as being limited thereto.

### [Example 1] Cloning of full length cDNA (ORF portion) of human FGF23

Cloning of the full-length cDNA (ORF portion) of FGF-23 was carried out by the PCR method. Primers were designed based on GenBank data (Accession No. AB037973) by adding an *EcoRI* site and XhoI site to the sequence (5'-ggAATTCTCgAgCCACCATgTTgggggCCCgCCTCAggCTCTg-3' /SEQ ID NO: 3; and 5'-ggAATTCTCgAgCTACTAgATgAACTTggCgAAgg-3'/SEQ ID NO: 4). Furthermore, Kozak sequence (CCACC) was added upstream of the ATG initiation codon for the 5'-side primer, and two TGA stop codons were added to the 3'-side sequence. Primer production was contracted out to Sawady Technology. Human heart cDNA (Multiple cDNA kit, Cat. No. CH-1101, OriGene) was used as the template and QIAGEN PCRkit (Cat. No. 201223, QIAGEN) was used for the PCR reaction. More specifically, 0.75 µL of human heart cDNA (Multiple cDNA kit, OriGene), 2.5 µL of QIAGEN 10x PCR buffer, 0.5 µL of dNTP mix (200 mM), 0.25 µL of QIAGEN Taq DNA polymerase, 5.0 µL of 5x Q-solution, 0.5 µL of Specific Forward PCR primer (50 µM, SEQ ID NO: 3) , 0.5 µL of Specific Reverse PCR primer (50 µM, SEQ ID NO: 4), and 15 µL of deionized distilled water (DDW) was mixed to a total volume of 25 µL for the PCR reaction which was carried out using thermal cycler ABI2400 under the following conditions: primary denaturation at 95°C for 2 min, 35 cycles of 2 steps shuttle PCR (at 94°C for 40 sec, then at 60°C for 1 min), and finally, elongation reaction at 72°C for 7 min to complete the PCR reaction. Then the amplified product of the PCR reaction was confirmed by 1.0% agarose gel electrophoresis. As a result, a single specific band was confirmed near 750 bp. Thus, subcloning of the amplified DNA into a TA vector pCR2.1 was carried out using TOPO TA Cloning kit (Cat. No. K45000-01, Invitrogen) using 2 µL of the PCR reaction solution. The procedure followed the protocol provided with the kit. Furthermore, nucleotide sequence analysis of clone #3 that had been cloned by the TA cloning was carried out using M13 M4 primer (Cat. No. 3832A, TaKaRa) , M13 RV primer (Cat. No. 3830A, TaKaRa), primer of SEQ ID NO: 5 (5'-CgCACCCCATCAgACCATCT-3') , and primer of SEQ ID NO: 6 (5'-gCAgTTCTCCgggTCgAAATA-3') using ABI377 DNA sequencer. As a result, the internal sequence of clone #3 was revealed to completely match to that of the human FGF-23. Finally, cloning of FGF-23 was accomplished.

### [Example 2] Production of human FGF mutants (R176Q, R179Q, R179W, R176Q+R179Q, and R176Q+R179W)

Using human FGF-23 as the template, FGF-23 mutants R176Q(M1), R179Q (M2), R179W (M3), R176Q+R179Q (M4), and R176Q+R179W (M5) were produced. According to the literature ("Autosomal dominant hypophosphataemic rickets is associated with mutations in FGF23", Nature Genetics, Vol.26, p345-348, November 2000) , 3 types of mutants, 527G→A(P176Q) , 536G→A(R179Q) , and 535C→T(R179W) , and combinations thereof, M4 (R176Q+R179Q) and M5 (R176Q+R179W), were produced from the human FGF-23. Primer synthesis was contracted out to Sawady Technology. The nucleotide sequences of the used primers were as follows: 5'- CACggCAgCACACCCggAgC -3' (SEQ ID NO: 7); 5'- CACggCggCACACCCAgAgC -3' (SEQ ID NO: 8); 5'- CACggCggCACACCTggAgC -3' (SEQ ID NO: 9); 5'- CACggCAgCACACCCAgAgC -3' (SEQ ID NO: 10); and

5'- CACggCAgCACACCTggAgC -3' (SEQ ID NO: 11).

Mutagenesis was carried out by a method comprising of 3 steps: producing a partial fragment for mutagenesis during the first PCR, producing a template of a full-length mutant containing the mutation (s) in the second PCR, and finally obtaining a complete mutant in the third PCR. More specifically, 0.2 µL of human FGF-23 clone #3 (40 ng/ µL) 5 µL of TaKaRa EX 1O PCR buffer, 4 µL of dNTP mix (50 µM) , 0.5 µM TaKaRa ExTaq DNA polymerase, 0.5 µL of Specific mutant primer (50 µM, SEQ ID NOs: 7, 8, 9, 10, or 11), 0.5 µL of Specific Reverse PCR primer (50 µM, SEQ ID NO: 4) , and 39.3 µL of DDW were mixed to a total volume of 50 µL to perform a PCR reaction using thermal cycler ABI2400 under the following conditions: primary denaturation at 95°C for 2 min, 35 cycles of 2 steps shuttle PCR (at 94°C for 40 sec, then at 60°C for 30 sec), and finally, elongation reaction at 72°C for 4 min to complete the PCR reaction. Then, the amplified product of the PCR reaction was confirmed by 1.0% agarose gel electrophoresis. As a result, a single specific band was confirmed near 200 bp. From this gel, each of the specific amplified fragments (R176Q and R179Q) was purified using QIAquick Gel Extraction Kit (Cat. No. 28704, QIAGEN) following the protocol provided with the kit. Next, using the purified fragments (partial sequences of the mutants), second PCR reactions (production of templates of the full-length mutants) were performed. Using 1 µL of the purified fragment as the primer, 0.1 µL of human FGF-23 clone #3 (40 ng/µL) , 2.5 µL of TaKaRa EX 10x PCR buffer, 2 µL of dNTP mix (50 µM) , 0.25 µL of TaKaRa ExTaq DNA polymerase, and 18.15 µL of DDW were mixed to a total volume of 24 µL to carry out the second PCR reaction using thermal cycler ABI2400 under the following conditions: primary denaturation at 95°C for 2 min, and 5 cycles of 3 step cycle PCR (at 94°C for 1 min, at 60°C for 1 min, and then at 72°C for 1 min) to complete the second PCR. Then, 0.5 µL of Specific Forward PCR primer (50 µM, SEQ ID NO: 3) and 0.5 µL of Specific Reverse PCR primer (50 µM, SEQ ID NO: 4) were added to this reaction solution to a total volume of 25 µL to conduct the third PCR reaction using thermal cycler ABI2400 under the following conditions: primary denaturation at 95°C for 2 min, 35 cycles of 2 steps shuttle PCR (at 94°C for 40 sec, then at 60°C for 1 min), and finally, elongation reaction at 72°C for 7 min to complete the PCR reaction. Next, the finally amplified product of the PCR reactions was confirmed by 1.0% agarose gel electrophoresis to show a specifically amplified band near 750 bp.

According to methods similar to that of Example 1, the subsequent analyses were carried out. Specifically, TA cloning of the internal sequences followed by nucleotide sequence analyses of the internal sequences was performed. As a result, clones of respective mutants containing the desired mutations were successfully obtained.

### [Example 3] Production of expression vectors

Expression vectors of each of the clones were produced by inserting the respective internal sequence of FGF-23 and each type of the mutants (M1 to M5) into pCAGGS3. More specifically, each clone was excised with *Eco*RI restriction enzyme, and the obtained *Eco*RI fragment was purified using QIAquick Gel Extraction Kit (Cat. No. 28704, QIAGEN) to be inserted into *Eco*RI cleaved pCAGGS3 . The vectors were named pCGF23, and pCGFM1 to pCGFM5, respectively.

### [Example 4] Preparation of endotoxin free plasmids

For direct *in vivo* administration of plasmid DNAs, plasmid purification was performed with additional endotoxin removal treatment. More specifically, pCGF23, and pCGFM1 to pCGFM5 were purified as endotoxin-free plasmids using Endofree plasmid Maxi Kit (Cat. No. 12362, QIAGEN) according to the protocol provided therewith.

### [Example 5] Addition of C-terminal FLAG-tags to FGF-23 and M2 (R179Q)

Mutants containing a FLAG sequence at the C-terminus were produced using pCGF23 and pCGFM2 as templates, with primer of SEQ ID NO: 12 comprising the sequence of SEQ ID NO: 3 and the FLAG sequence. More specifically, 1 µL of pCGF23 or pCGFM2 (30 ng/µL) , 2 . 5 µL of TaKaRa EX 10x PCR buffer, 2 µL of dNTP mix (50 µM) , 0.25 µL of TaKaRa ExTaq DNA polymerase, 0.5 µL of Specific Forward PCR primer (50 µM, SEQ ID NO: 11), 0.5 µL of Specific Reverse PCR primer (50 µM, 5'-ggATCCgAATTCATATgTCACTTATCgTCgTCATCCTTgTAATCgATGAACTTggCgAAgg - 3'/SEQ ID NO: 12), and 18.5 µL of DDW was mixed to a total volume of 25 µL to conduct a PCR reaction using thermal cycler ABI2400 under the following conditions: primary denaturation at 95°C for 2 min, 30 cycles of 2 steps cycle PCR (at 94°C for 30 sec, then at 60°C for 1 min) , and finally, elongation reaction at 72°C for 7 min to complete the PCR reaction. Then the finally amplified product from the PCR reaction was confirmed by 1.0% agarose gel electrophoresis to show a specifically amplified band near 800 bp. Subsequent analyses were carried by a similar method to Examples 1 to 4, and ultimately, expression vectors pCGF23-F and pCGFM2-F were produced.

### [Example 6] Addition of N-terminal FLAG-tags to FGF-23 and M2 (R179Q)

An FGF23 expression vector carrying an N-terminal FLAG-tag was constructed as described below by the PCR method. First stage PCR reaction (25 cycles of 96°C for 15 sec, 55°C for 15 sec, and 72°C for 2 min) was performed using 3 ng of pCG23 as the template, 100 pmol each of Specific Forward PCR primer and 23N FLAG R (GCCCTTATCGTCGTCATCCTTGTAATCGGCTCTGAGGACGCTC/SEQ ID NO: 13) , or 23R1 (GGCTCGAGTCAGATGAACTTGGCGAAGG/SEQ ID NO: 14) and 23N FLAG F (GATGACGACGATAAGGGCGGAGGTTCCAGAGCCTATCCCAATG/SEQ ID NO: 15) as the primer set, and TaKaRa ExTaq and the buffer provided therewith. After removing the primers from the PCR reaction products by filtration through Microcon-30 (Millipore) , a mixture of each of the PCR reaction products was used as the template, and FGF F and 23R1 were used as the primer set to carry out the second stage PCR reaction under the same conditions as in the first stage. After completion of the reaction, the PCR reaction products were purified by agarose gel electrophoresis. The fragment was cloned using TOPO Cloning kit (Invitrogen) and its DNA sequence was determined to confirm the introduction of the desired mutation without unnecessary mutations. The plasmid with the confirmed sequence was prepared, cleaved with *Eco*RI, and generated fragment was collected to inserte it into pCAGGS3 that had been cleaved with EcoRI. Finally, after confirming the direction of the inserted fragment, the expression vector was dubbed pCGF23NF.

An FGF23M2 vector carrying a N-terminal FLAG-tag was produced according to the same method as described above, except that pCGFM2 was used as the template for the first stage PCR, and was dubbed pCGFM2NF.

### [Example 7] Production of expression vectors for naked DNA injection experiment in animals

Using the naked DNA injection method, FGF23 and mutants thereof were examined whether they directly or indirectly influence the phosphorus metabolism in adult mice.

Materials as follows were used:
FGF23 expression vector (pCGF23)
R176Q FGF23 mutant expression vector (pCGFM1)
R179QFGF23 mutant expression vector (pCGFM2)
R179WFGF23 mutant expression vector (pCGFM3)
R176QR179QFGF23 mutant expression vector (pCGFM4)
R176QR179WFGF23 mutant expression vector (pCGFM5)

### <Control substance (negative control substance)>

MOCK vector (pCAGGS)

Form: 10 mM Tris/1 mM EDTA (pH 8.0) solution

Storage: -20°C, in the dark

### <Method of preparation>

The dosage form was a solution, and the method of preparation followed the protocol of *Trans*IT *In Vivo* Gene Delivery System (PanVera) *(Transit*® *In Vivo* Gene Delivery System (Pan Vera) standard protocol) . Ten µg of MOCK vector, FGF23 expression vector, or a FGF23 mutant expression vector was used for the administration to each animal. Ten µL of TransIT Polymer Solution and an appropriate amount of sterilized water were mixed to a final volume of 200 µL in a 50 mL Falcon tube. After leaving standing for 5 minutes at room temperature, 2.8 mL of 1x Delivery Solution was added to the 200 µL mixture mentioned above to give a total volume of 3.0 mL as a solution for administration. When administering to 6 animals, an amount corresponding to 7 animals, i.e., 21 mL, was prepared as the solution. The solution for administration was used up on the day of preparation.

Animals used in the experiment and their housing conditions are given below.

### <Used Animals>

Animal species: mouse
Lineage: Jcl: CD-1(ICR) or Crj: CD-1(ICR)
Sex: male
Weight: 35 g to 40 g
Age: 8 to 9 weeks at the time of administration
Supplier: CLEA Japan, or Charles River Japan
Method of euthanasia: bleeding under anesthesia
Acclimation period: approximately 2 weeks
Grouping method: random assigning

### <Breeding environment>

Room temperature: 24 ± 2°C
Relative humidity: 55 ± 10%
Ventilation frequency: 10 to 30 times/hour
Lighting time: 5:00 to 19:00
Feed: CE-2 (CLEA Japan) ad libitum
Drinking water: tap water ad libitum

### <Method of testing>

Intravenous administration was conducted with a dose of 3 mL, and the entire amount was administered within 8 seconds;

### <Sample collection>

### (1) Serum

On day 4 post-administration, whole blood was collected from the abdominal aorta under etherisation. The collected blood was placed in Separapid tube mini (Sekisui chemical) and was centrifuged (1,400x g, 10 min, 4°C) to separate serum. The serum was stored in a freezer set to -20°C until measurements.

### (2) Urine

On day 3 post-administration, mice were placed into glass metabolic cages (METABOLICA, SUGIYAMA-GEN IRIKI) and pooled 24-hour urine collection was made over the 4th day. The urine was stored in a freezer set to -20°C until measurements were made.

### (3) Kidney

After collecting the serum, both kidneys were removed, decapsulated, and subjected to the purification of brush border membrane vesicles.

### <Measurement of each parameter>

Inorganic phosphorus (Pi), calcium (Ca) , urea nitrogen (UN), and creatinine (CRE) in the serum or urine were measured with autoanalyzer (Hitachi 7170E model). 25-Hydroxyvitamin D, and 24,25-dihydroxyvitamin D were measured by a competitive protein binding assay (CPBA), and 1α,25-dihidroxyvitamin D was measured by the RIA2 antibody method.

### <Statistical analysis method>

Unpaired *t*-tests were performed between the MOCK vector administered group and the FGF23 expression vector administered group, or each of the FGF23 mutant expression vector administered groups. The significance levels were 5% (two tailed) . SAS Ver. 6.12 was used as the analysis software.

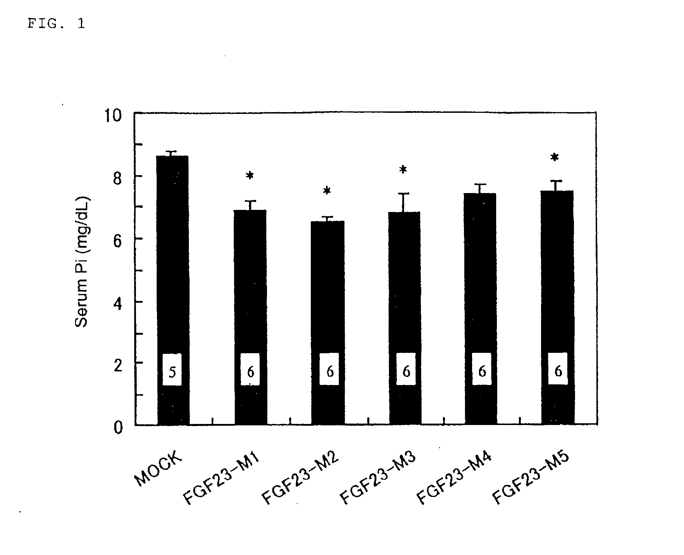

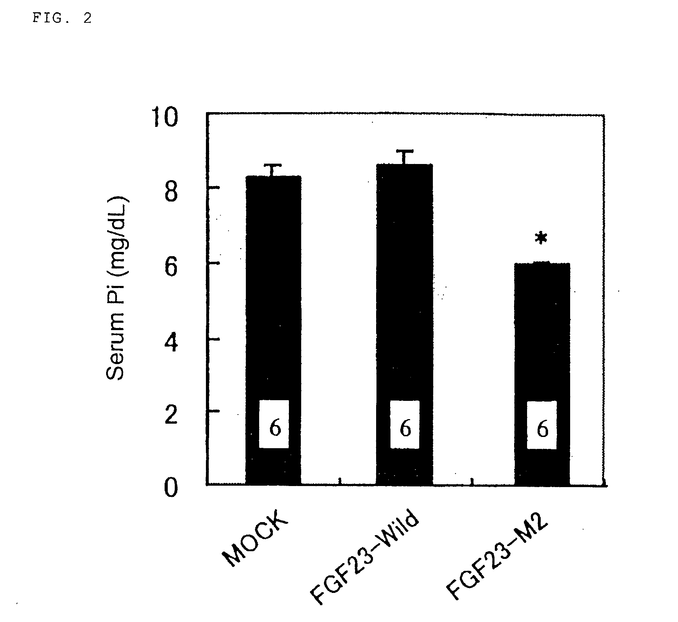

As a result, regarding the effect on serum biochemistry, significant decrease in the blood phosphorus level compared to the MOCK administered group was observed, regardless of the type of mutant, in the groups administered with either of the 3 types of FGF23 mutant expression vectors introduced with a point mutation identified in Autosomal dominant hypophosphatemic rickets (ADHR) patients (i.e., FGF23-M1, FGF23-M2, or FGF23-M3) as shown in Fig. 1. However, although a similar serum phosphorus decreasing effect was observed in groups administered with either of the double mutant expression vectors having a combination of these point mutations (i.e., FGF23-M4 or FGF23-M5) , no additive or synergistic effect was confirmed because of combining the point mutations. In the group administered with the wild type FGF23 (FGF23-Wild), no serum phosphorus decreasing effect was observed (Fig. 2).

As shown in Table 1, no significant differences were observed in other serum biochemical parameters (calcium, creatinine, and urea nitrogen) between the MOCK group and the FGF23-Wild group or the FGF23-M2 group.

**Table 1**

| | Inorganic phosphorus (mg/dL) | Calcium (mg/dL) | Creatinine (mg/dL) | Urea nitrogen (mg/dL) |
|---|---|---|---|---|
| MOCK | 8.3 ±0.3 | 9.2 ± 0.2 | 0.34 ±0.01 | 22.7 ± 1.4 |
| FGF23-Wild | 8.6 ± 0.4 | 9.4 ± 0.1 | 0.41 ± 0.06 | 31.1 ± 3.7 |
| FGF23-M2 | 6.0 ± 0.1 | 8.9 ± 0.1 | 0.33 ± 0.01 | 23.4 ± 1.3 |

Furthermore, regarding the result indicating the effect of mutants on urine biochemistry, as shown in Table 2, a tendency toward increased phosphorus excretion level per day was observed in the FGF23-M2 administered group compared to the MOCK group, however, not with a significant difference.

**Table 2**

| | Inorganic phosphorus excretion level (mg/day) | Calcium excretion level (mg/day) |
|---|---|---|
| MOCK | 3.29 ± 0.87 | 0.07 ± 0.02 |
| FGF23-M2 | 4.79 ± 0.92 | 0.16 ± 0.01 |

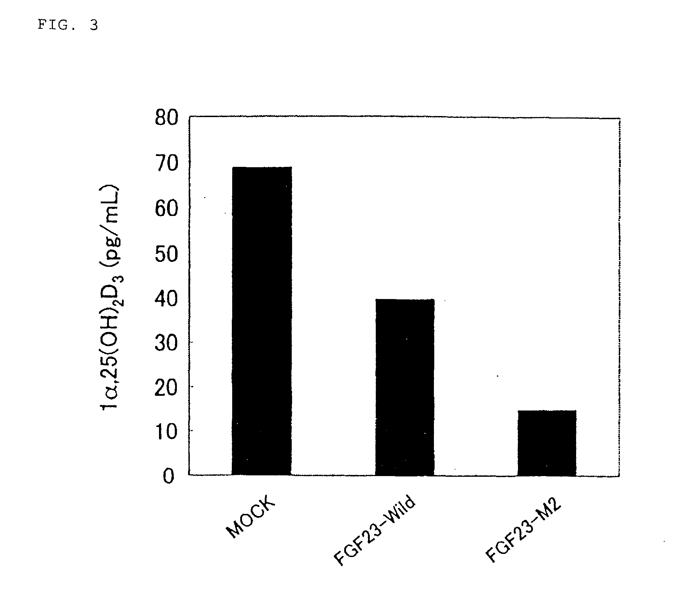

Furthermore, regarding the result indicating the effect of mutants on serum 1α,25(OH)₂D₃, as shown in Fig. 3, the 1α, 25(OH)₂D₃ level significantly decreased in the FGF23-M2 administered group and FGF23-Wild group compared to the MOCK administered group. The 1α,25(OH)₂D₃ levels decrease to approximately half in the FGF23-Wild group, and to below measurement sensitivity in the FGF23-M2 administered group.

On the other hand, no significant difference was confirmed in the level of the *in vivo* precursor, 25(OH)D₃, among the MOCK administered group, FGF23-Wild administered group, and FGF23-M2 administered group. Furthermore, although no difference was observed in the 24,25 (OH) ₂D₃ level between the FGF23-Wild administered group and the MOCK administered group, remarkable decrease was observed in the FGF23-M2 administered group (Table 3).

**Table 3**

| | 25-hydroxyvitamin D₃ (ng/mL) | 24,25-dihydroxyvitamin D₃ (ng/rnL) |
|---|---|---|
| MOCK | 20.8 | 14.1 |
| FGF23-Wild | 25.2 | 12.0 |
| FGF23-M2 | 18.3 | 4.1 |

### <Purification of brush border membrane vesicles>

Purification of brush border membrane vesicles was performed by magnesium precipitation method. More specifically, ice-cooled MET buffer (60 mM mannitol, 1 mM EGTA, 2.5 mM Tris/HCl, pH7.1) was added to the collected kidney, following homogenization using PHYSCOTRON at 18,000 rpm for 1 minute, 1/10 volume of 1 M MgCl₂ was added, stirred, and then left standing on ice for 15 minutes. After removing unhomogenized fraction by low speed centrifugation (2,000x g, 15 min, 4°C), the supernatant was subjected to high speed centrifugation (24,000x g, 30 min, 4°C) to obtain precipitate. MET buffer was added to the precipitate and was further homogenized with Teflon homogenizer (1,000 rpm, 10 strokes). Again, 1/10 volume of 1 M MgCl₂ was added, stirred, kept on ice for 15 minutes, and following centrifugation of the mixture at low speed (2,000x g, 15 min, 4°C), the supernatant was centrifuged at high speed (24,000x g, 30 min, 4°C). Transport Buffer-K (100 mM mannitol, 20 mM HEPES/Tris, pH7.4) was added to the obtained precipitate, and brush border membrane vesicles were obtained by repeating suction and discharge with a plastic syringe (20G and 27G needles).

### <Measurement of phosphorus transport activity>

Rapid filtration method was used for measuring phosphorus transport activities using brush border membranes. More specifically, 20 µL of the brush border membrane vesicles and 80 µL of reaction solution (100 mM mannitol, 20 mM HEPES/Tris, pH 7.4, 125 mM NaCl, 125 nM ³²P-KH₂PO₄) were reacted for 1 minute at 25°C, and 1 mL of quenching solution (100 mM mannitol, 100 mM choline chloride, 20 mM MgSO₄, 5 mM KH₂PO₄, 20 mM HEPES/Tris, pH 7.4) was added to stop the reaction. Then, the reaction solution was subjected to suction filteration through a nitrocellulose membrane (pore size 0.45 µm, 2.5 cm diameter) and the nitrocellulose membrane was washed with 5 mL of the quenching solution. The radioactivity trapped on the membrane was measured with liquid scintillation counter TRI-CARB 2700TR (Beckman) as total phosphorus transport activity. Sodium independent phosphorus transport activity was measured by substituting 125 mM KC1 for 125 mM NaCl in the reaction solution. Sodium dependent phosphorus transport activity was calculated as the difference between the total phosphorus transport activity and the sodium independent phosphorus transport activity. Both activities are expressed as the amount of phosphorus absorbed by a unit of protein in 1 minute (pmoles/mg protein/min) by measuring the amount of protein in the brush border membrane vesicles with BCA Protein Assay Reagent (PIERCE).

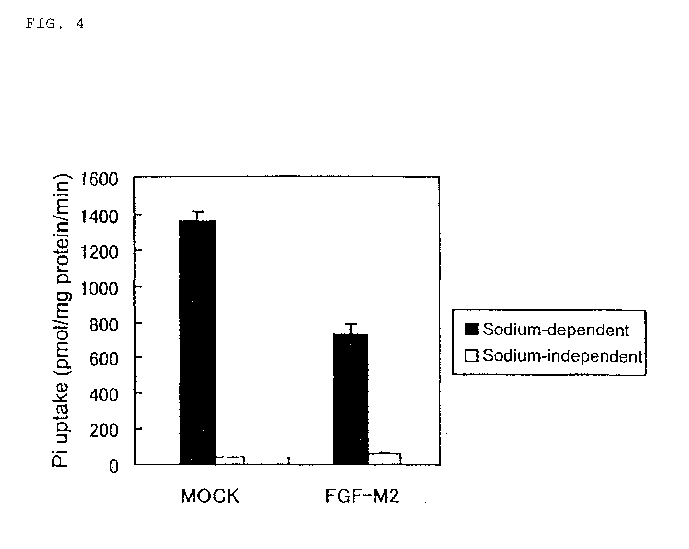

As a result, as shown in Fig. 4, the sodium dependent phosphorus transport carrier (Na/Pi) activity of kidney was significantly decreased in the FGF23-M2 administered group compared to the MOCK administered group. On the other hand, no changes were found in the sodium independent phosphorus transport activity.

### [Example 8] Construction of C-terminus deleted M2FGF23 mutant expression vector

C-terminus deleted M2FGF23 mutant expression vector was constructed using the PCR method as described below. PCR reaction (25 cycles of 96°C for 15 sec, 55°C for 15 sec, and 72°C for 2 min) was carried out using TaKaRa ExTaq and the buffer provided therewith, with 3 ng of pCGFM2NF as the template, and 100 pmol each of Specific Forward PCR primer and dC188 (GGCTCGAGTCAGTCCCGCTCCGAGTC/SEQ ID NO: 16) , dC194 (GGCTCGAGTCACTTCAGCACGTTCAGGGG/SEQ ID NO: 17), dC200 (GGCTCGAGTCAGGTCATCCGGGCCCGGGG/SEQ ID NO: 18) , dC210 (GGCTCGAGTCAGAGCTCCTGTGAACAGGA/SEQ ID NO: 19) , dC217 (GGCTCGAGTCAGCTGTTGTCCTCGGCGCT/SEQ ID NO: 20) , dC223 (GGCTCGAGTCAGTCACTGGCCATCGGGCT/SEQ ID NO: 21), dC240 (GGCTCGAGTCAGCCCGTTCCCCCAGCGTG/SEQ ID NO: 22), or dC245 (GGCTCGAGTCAGCGGCAGCCTTCCGGGCC/SEQ ID NO: 23) as the primer set. After the termination of the reaction, the PCR reaction products were purified by agarose gel electrophoresis. Obtained fragments were cloned using TOPO Cloning kit (Invitrogen). Then, their DNA sequences were determined to confirm that desired mutations are introduced without unnecessary mutations. Plasmids with confirmed sequences were prepared, following cleavage with ECORI, fragments were collected and inserted into pCAG GS3 that had been cleaved with EcoRI. Expression vectors thus obtained were dubbed pCGdC188 (dC188), pCGdC194 (dC194), pCGdC200 (dC200), pCGdC210 (dC210), pCGdC217 (dC217), pCGdC223 (dC223), pCGdC240 (dC240), and pCGdC245 (dC245), respectively.

### [Example 9] Expression of C-terminus deleted M2FGF23 mutant protein

1x 10⁵ COS cells were suspended in 1 mL of DMEM (10% FCS) media (GIBCO), and were plated onto a 6-well plate. After culturing overnight, 1 µg of the expression vector (pCGdc188 to pCGdc245 shown in Example 8) was transfected into the cells using 3 µL of FuGene (Boeringer) , and the cells were further cultured overnight. On the following day, the media was replaced with CHO-S-SFMII, and cultivation was continued for another 2 days. Two days later, the media was collected, and mutant protein expressed in the media was analyzed by Western Blotting using anti-FLAG antibody (M2) (SIGMA) to confirm the expression of the mutant protein.

### [Example 10] Transient expression of the recombinant FGF-23 mutant (M2) in COS cells

5x 10⁶ COS cells were suspended in 400 µL of DMEM (10% FCS) media (GIBCO), 10 µg of pCGFM2-F expression vector was added, and was transferred into a 0.4 cm electroporation cuvette (BIO-RAD Laboratories). Electroporation was carried out using Gene-pulser (BIO-RAD Laboratories) under conditions of: 0.26 kV, resistance∞, and 960 mF. Subsequently, the cell solution was suspended in 30 mL of DMEM (10% FCS) media, transferred into a 175 cm² flask (FALCON), and was left standing for a whole day and night at 37°C in a CO₂ incubator (ESPEC). The following day, the media was replaced with 30 ml of CHO-S-SFMII media (GIBCO), and the cells were further cultured for 3 days. The collected media was centrifuged at 3,000 rpm for 15 minutes to remove the cells. Then, the media was analyzed by Western Blotting using anti-FLAG antibody (M2) (SIGMA) to confirm the expression of the recombinant FGF-23 mutant (M2)-F.

### [Example 11] Purification of recombinant FGF-23(M2)-F using affinity column

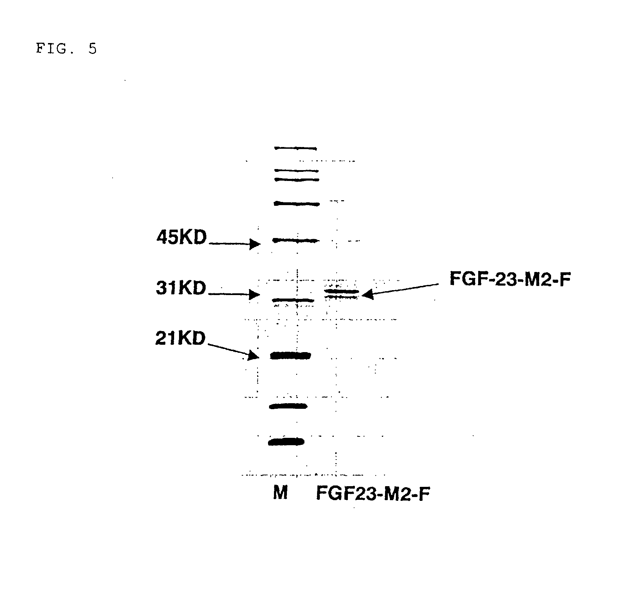

Purification of recombinant FGF-23 mutant (M2)-F was performed using anti-FLAG antibody affinity column (SIGMA). The chromatography procedure was carried out using GradiFrac System (Pharmacia). After equilibrating the affinity column with PBS-T, the media containing the expressed recombinant FGF-23 mutant (M2)-F prepared in Example 10 was applied to the column, and was eluted with glycine HCl buffer (pH 3.5) . Then, the main peak was fractioned and analyzed by SDS-PAGE. Because a nearly uniform band near the desired molecular weight was confirmed by Coomassie brilliant blue (CBB) staining, the preparation of the recombinant was terminated (Fig. 5).

### [Example 12] Deletion mutation experiment on animals

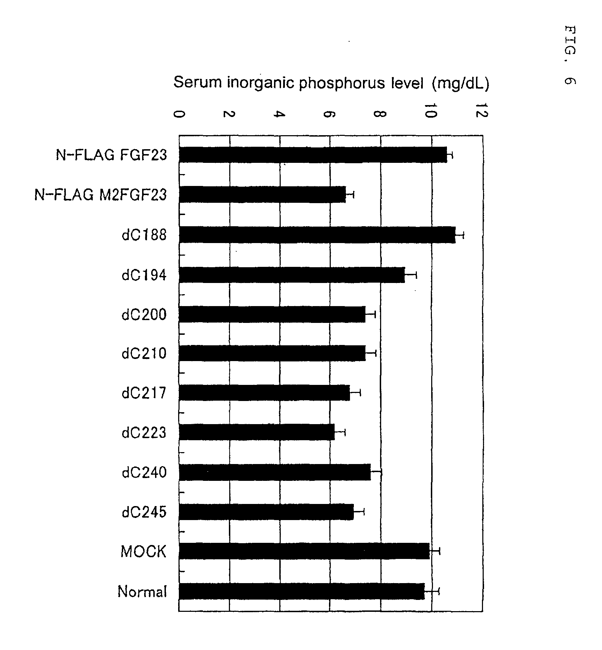

Preparation of administration agent was conducted following the protocol of *Trans*IT *In Vivo* Gene Delivery System (PanVera). 10 µL of *Trans*IT Polymer Solution and an appropriate amount of sterilized water were added to 10 µg of respective expression vectors shown in Fig. 6 (MOCK is the same as that in Example 7) to a total volume of 200 µL. The solution was mixed, left standing at room temperature for 5 minutes, and 2.8 mL per 200 µL of 1x Delivery Solution was added to yield an administration solution for each animal with a total volume of 3.0 mL. When administering to 6 animals, an amount for 7 animals, i.e., 21 mL, was prepared as the solution. The solution for administration was used up on the day of preparation.

Eight to 9-week old female CD-1 (ICR) mice (35 g to 40 g) purchased from Charles River Japan were subjected to experiments after 1-week acclimation. The entire 3 mL of the administration agent containing the expression vector was administered within 8 seconds from the tail vein. Four days after administration, whole blood was collected from the abdominal aorta under etherisation. The collected blood was placed in Separapid tube mini (sekisui Chemical) and was centrifuged (1,400x g, 10 min, 4°C) to separate the serum.

Inorganic phosphorus (Pi), calcium (Ca) , urea nitrogen (UN), and creatinine (CRE) in the serum were measured using an autoanalyzer (Hitachi 7170E model).

The results are shown in Fig. 6.

The M2FGF23 mutant is composed of 251 amino acids. When the C-terminal portion of the mutant is shortened, a mutant, dC200, having the amino acids up to position 200 also had a similar serum phosphorus decreasing effect. However, when the C-terminus is further shortened to a dC194 mutant containing the amino acids up to position 194, or a dC188 mutant containing the amino acids up to position 188, the effect was weakened and lost. It has been supposed that in ADHR patients, mutation of the amino acid at position 176 or position 179 makes FGF23 less susceptible to regulation by proteolysis, and consequently existence of excess FGF23 in blood leads to hypophosphatemia. Therefore, according to the present experimental result, the region of amino acid positions 179 to 200 was considered to include a site that is important for the serum phosphorus decreasing effect.

### [Experiment 13] The effect of FGF23 on thyroid-parathyroid-ectomized rats

An 8-week old male Sprague-Dawley (SD) rat was thyroid-parathyroid-ectomized (TPTX). Few days later, ionized calcium was measured to confirm the success of the operation. Then, a catheter was inserted into the femoral vein, urine sack was attached, and the rat was restrained in a Ballman cage.

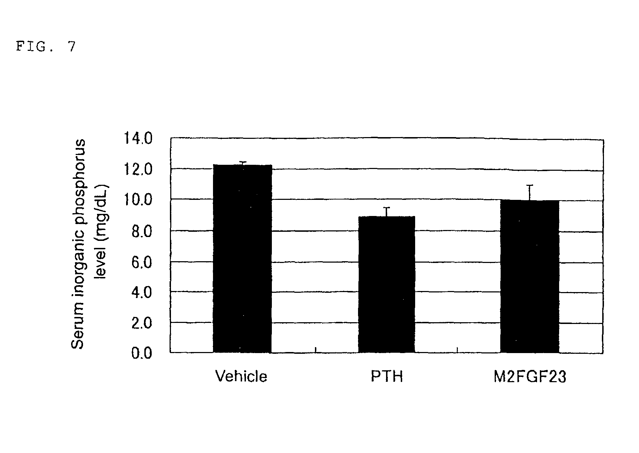

Using a Harvard Infusion Pump, PTH (1-34) (0.3 nmol/mL), M2FGF23 protein with a C-FLAG tag (6 µg/mL) , or vehicle (0.05% Tween 80/Saline) was administered by infusion at a rate of 1 mL/hr for 6 hours. Urine collection was taken within 4 hours to 6 hours after starting infusion until the termination of the administration. After the termination of the administration, whole blood was collected from the abdominal aorta. The collected blood was centrifuged at 3,000 rpm for 10 minutes, insoluble fractions of the serum and urine were separated, and inorganic phosphorus, calcium, and creatinine were measured using Hitachi 7170 model autoanalyzer. The results are shown in Figs. 7 to 9.

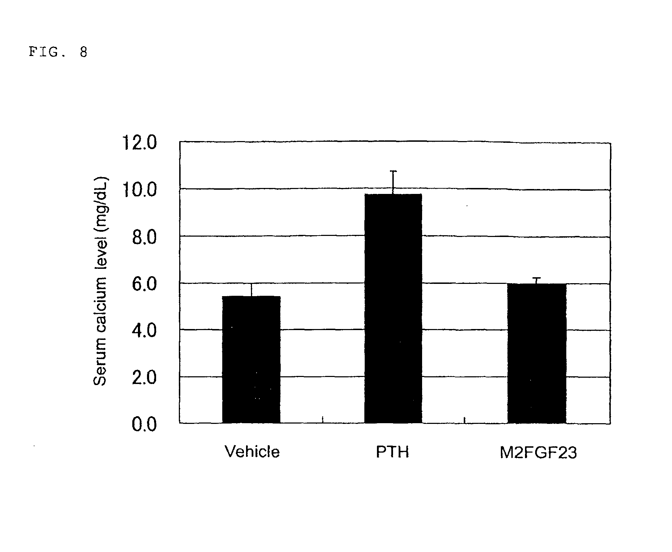

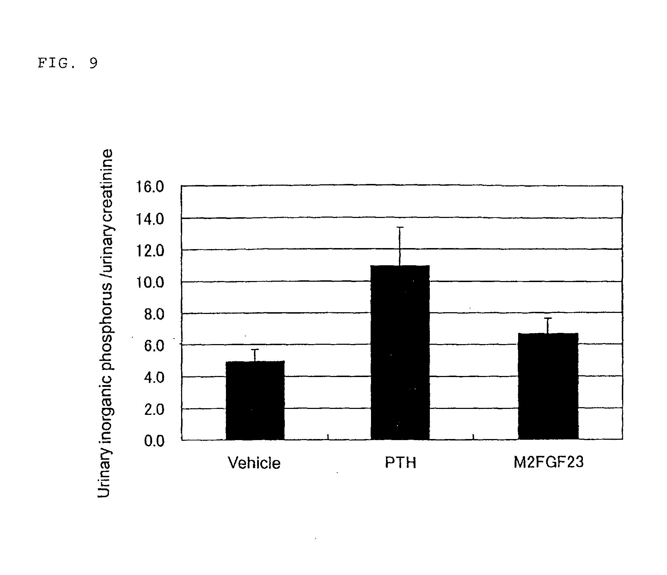

According to Fig. 7, the rat serum phosphorus concentration, which increased due to TPTX, normalized because of administration of PTH (1-34). Furthermore, similar normalization occurred by the administration of M2FGF23. On the other hand, although the decreased serum calcium concentration resulting from TPTX was corrected by the administration of PTH (1-34), M2FGF23 administration showed almost no effect (Fig. 8). It was revealed that the effect of M2FGF23 on serum phosphorus level was not caused via PTH, and thus does not affect the serum calcium level. Since the inorganic phosphorus/creatinine value of urine is increased through the PTH (1-34) administration, phosphorus in the serum is considered to be excreted into the urine. On the other hand, the inorganic phosphorus/creatinine value is unchanged by the administration of M2FGF23. Thus, phosphorus in the serum may have returned to the bone as hydroxyapatite (Fig. 9).

### Industrial Applicability

The present invention provides FGF23 protein mutants. Since the FGF23 mutants of the present invention have the effect to decrease the phosphorus level in the blood, they are expected to serve as therapeutic and preventive agents for hyperphospatemia. Furthermore, the DNAs encoding the FGF23 mutants of the present invention decrease the blood phosphorus level via their introduction and expression *in vivo*. Therefore, these DNAs of are expected to be applicable in gene therapy against hyperphosphatemia.

## Claims

1. A DNA encoding a protein comprising the amino acid sequence of SEQ ID NO: 2 having a mutation selected from the group of mutations of arginine at position 176 to glutamine, arginine at position 179 to glutamine, and arginine at position 179 to tryptophan.

2. A DNA encoding a fragment having at least the amino acid sequence from position 1 to position 190 of a protein comprising the amino acid sequence of SEQ ID NO: 2 that has a mutation selected from the group of mutations of arginine at position 176 to glutamine, arginine at position 179 to glutamine, and arginine at position 179 to tryptophan.

3. A vector into which the DNA according to claim 1 or 2 is inserted.

4. A transformed cell harboring the DNA according to claim 1 or 2, or the vector according to claim 3.

5. A protein comprising the amino acid sequence of SEQ ID NO: 2 having a mutation selected from the group of mutations of arginine at position 176 to glutamine, arginine at position 179 to glutamine, and arginine at position 179 to tryptophan.

6. A fragment having at least the amino acid sequence from position 1 to position 190 of a protein comprising the amino acid sequence of SEQ ID NO: 2 that has a mutation selected from the group of mutations of arginine at position 176 to glutamine, arginine at position 179 to glutamine, and arginine at position 179 to tryptophan.

7. A method for producing the protein according to claim 5 or 6, wherein the method comprises the steps of culturing the transformed cell according to claim 4, and collecting expressed protein from the transfected cell or the culture supernatant thereof.

8. A pharmaceutical composition for decreasing the blood phosphorus level, which comprises the DNA according to claim 1 or 2, the vector according to claim 3, or the protein according to claim 5 or 6.

9. The pharmaceutical composition according to claim 8, which does not influence the blood calcium level.

10. The pharmaceutical composition according to claim 8 or 9 for treating hyperphosphatemia.

11. A method for treating hyperphosphatemia, which comprises the step of administering the DNA according to claim 1 or 2 to a patient.
